# EUROPÄISCHE PATENTSCHRIFT

(11) **EP 0 667 806 B1**
(45) Veröffentlichungstag und Bekanntmachung des Hinweises auf die Patenterteilung: **13.03.1996**
(21) Anmeldenummer: 94900093.9
(22) Anmeldetag: 05.11.1993
(51) Int. Cl.: B01J 23/94, B01J 23/92, B01J 38/00, B01J 37/00

(54) **VERFAHREN ZUR HERSTELLUNG VON EISEN, KALIUM UND CER ENTHALTENDEN KATALYSATOREN**
METHOD OF PRODUCING CATALYSTS CONTAINING IRON, POTASSIUM AND CERIUM
PROCEDE DE FABRICATION DE CATALYSEURS CONTENANT DU FER, DU POTASSIUM ET DU CERIUM

(30) Priorität: 09.11.1992 DE 4237740
(43) Veröffentlichungstag der Anmeldung: 23.08.1995
(73) Patentinhaber: BASF Aktiengesellschaft, D-67063 Ludwigshafen (DE)
(72) Erfinder: POEPEL, Wolfgang, Juergen, D-64297 Darmstadt (DE); TREMMEL, Gregor, D-67269 Gruenstadt (DE); BUECHELE, Wolfgang, D-67063 Ludwigshafen (DE); DEIMLING, Axel, D-67434 Neustadt (DE); PETERSEN, Hermann, D-67269 Gruenstadt (DE)
(86) Internationale Anmeldenummer: EP9303083
(87) Internationale Veröffentlichungsnummer: WO9411104

(56) Entgegenhaltungen:
- EP-A- 0 043 684
- EP-A- 0 452 630
- EP-A- 0 550 861
- DE-A- 4 006 918
- US-A- 4 888 316

## Beschreibung

Die Regenerierung von Katalysatoren ist ein sehr altes Anliegen der Katalysatorforschung, da gebrauchte Katalysatoren regelmäßig eine wertvolle Quelle für die in ihnen enthaltenen Elemente bzw. deren Verbindungen sind. Aus Gründen des Umweltschutzes ist auch eine Ablagerung, d.h. Verzicht auf Regenerierung nicht mehr zu vertreten, selbst in den Fällen, in denen sie aus wohlfeilen Bestandteilen wie Eisen- oder Aluminiumoxid bestehen.

Es gibt daher schon sehr viele - z.T. auch sehr alte - Vorschläge zur Rückgewinnung oder Wiederaufarbeitung des Katalysatormaterials.

Grundsätzlich unterscheidet man zwischen einer formerhaltenden und einer neuformenden Regenerierung. Während im ersteren Falle lediglich Verunreinigungen (z.B. Ruß o.ä.) entfernt - vgl. US-PS 2 831 041 (1954) - und gegebenenfalls fehlende Bestandteile z.B. durch Tränken mit einer entsprechenden Lösung des Bestandteils ersetzt werden, wird - vor allem bei trägerfreien, an sich langlebigen Katalysatoren oft die Neuformung vorgezogen.

Hierbei hat man ursprünglich - vgl. US-Patent 2 666 086 (1949) - bei Dehydrierungskatalysatoren, die traditionell im wesentlichen aus Eisenoxid bestehen, den Katalysator als Ganzes in einer Säure gelöst, die Zusammensetzung neu eingestellt und dann die Lösung in üblicher Weise gefällt oder eingedampft und aus dem erhaltenen Feststoff den gewünschten Katalysator neu geformt.

Nun ist es aber wünschenswert, auf die chemische Neuschaffung der wirksamen Bestandteile (d.h. Auflösen und Wiederausfällen) zu verzichten.

Die Erfindung hat sich die Aufgabe gestellt, ein Verfahren zur Herstellung von Eisen, Kalium und Cer enthaltenden Katalysatoren (vgl. EP 195 252; DE-Patent 28 15 874) für die Dehydrierung von Kohlenwasserstoffen aus ebensolchen gebrauchten Katalysatoren (Regenerierung) durch Zerkleinern, z.B. durch Vermahlen des gebrauchten Materials, gegebenenfalls Reinigung, Wiederherstellen der ursprünglichen Wirkung durch Einstellen der Zusammensetzung und Wiederherstellen einer äußeren Form anzugeben, das einen neuwertigen Katalysator liefert, ohne daß eine chemische Umsetzung erforderlich ist. Es hat sich dabei gezeigt, daß das Wiederherstellen der ursprünglichen Zusammensetzung im speziellen Falle der Cer enthaltenden Katalysatoren nicht ausreicht, um die ursprüngliche Aktivität wiederzuschaffen.

Erfindungsgegenstand ist daher ein Verfahren der vorgenannten aufgabengemäßen Art, das im wesentlichen darin besteht, dem zerkleinerten z.B. vermahlenen Material eine wirksame Menge Kalium und eine solche Menge an Cer zuzusetzen, daß die Gesamtmenge an Cer höher als die ursprünglich vorhandene Menge ist.

Daneben können natürlich auch andere Bestandteile, wie sie üblicherweise in Fe-K-Ce-Katalysatoren mitverwendet werden, wieder auf ihren ursprünglichen oder einen anderen, wünschenswerten Gehalt gebracht werden. Katalysatoren dieser Art sind in den vorgenannten Druckschriften (EP-, DE-Patent) beschrieben, auf die verwiesen wird.

Die Erfindung beruht auf der überraschenden Erkenntnis - die die Erfindung nicht begrenzen soll - daß speziell Cer in den erfindungsgemäßen Katalysatoren keineswegs vollständig ausgetragen wird, also verschwindet, sondern lediglich in eine Form übergeht, die durch Zerkleinern, z.B. Vermahlen allein nicht regenerierbar ist.

Daher muß die analytische Zusammensetzung hinsichtlich des Cer-Anteils korrigiert werden; während frische Katalysatoren neben überwiegend (z.B. 40 bis 90, insbesondere 70 bis 90 Gew.-%) Eisenoxid (Fe₂O₃) etwa 5 bis 40 (insbesondere 5 bis 20) Gew.-% Kalium, 0,1 bis 20 (insbesondere 1 bis 20) Gew.-% Cer und eventuell weitere Bestandteile enthalten, wobei die Gesamtmenge der Katalysatorbestandteile 100 Gew.-% beträgt, sollte ein regenerierter Katalysator neben den anderen, in entsprechend geringerer Menge vorhandenen Bestandteilen nunmehr 2 bis 40 Gew.-% Cer aufweisen. Weitere Regenerierung, d.h. Wiederholung der Regenerierung eines bereits ein- oder mehrfach regenerierten Katalysators ist möglich, wobei der Cer-Gehalt rein analytisch weiter ansteigt. Dies ist für die Wirkung ohne Belang, weiß man doch, daß auch Katalysatoren, die überwiegend, d.h. von vornherein, aus Ceroxid bestehen, ohne weiteres zum Dehydrieren verwendet werden können. Es versteht sich, daß ein mehrfach erfindungsgemäß regenerierter Katalysator schließlich immer noch durch Auflösen, Wiedergewinnung der enthaltenen Elemente und Neuherstellung verwertet werden kann, wobei ggf. die ursprüngliche Zusammensetzung wieder eingestellt werden kann. Ebenso versteht es sich, daß ein regenerierter Katalysator durch frischen Katalysator ergänzt werden kann, z.B. wenn eine größere Menge als ursprünglich vorhanden gebraucht wird.

### Beispiele

Zur Beurteilung der Leistung eines Dehydrierkatalysators wird einerseits die Selektivität, andererseits die Aktivität herangezogen.

Selektivität ist die Fähigkeit des Katalysators, die gewünschte Reaktion zu fördern und unerwünschte Reaktionen zu unterdrücken bzw. nicht zu fördern. Sie wird im Falle der Umsetzung von Ethylbenzol zu Styrol ermittelt durch die Bestimmung des Verhältnisses der erzeugten Styrolmasse zu der umgesetzten Masse an Ethylbenzol und angegeben in Molprozenten (anderweitig auch als Ausbeute bezeichnet, was aber zu Verwechslungen führen kann, da oft auch der Umsatz, d.h. der Umsatz pro Durchgang so bezeichnet wird).

Aktivität ist die Leistung des Katalysators im Bezug auf die Umsetzungstemperatur. Sie wird - unter vergleichbaren Bedingungen - gemessen und angegeben als die Temperatur, bei der ein bestimmter Umsatz (pro Durchgang) erzielt wird; im vorliegenden Fall wird der Umsatz an Ethylbenzol auf 70 Mol-% eingestellt. Dieser ergibt sich, indem die Umsetzungstemperatur (Temperatur einer den Reaktor umgebender Wärmequelle wie eine Elektroheizung oder ein Salzbad) variiert wird. Es versteht sich somit, daß höhere Aktivität durch eine niedrigere Reaktionstemperatur angezeigt wird und umgekehrt.

Praktisch wurden jeweils 1000 g Katalysator in ein 30 mm weites und 1200 mm langes Stahlrohr eingefüllt, das mit einer heiz- und regelbaren Elektroheizquelle umwickelt und mit üblichen Ausrüstungsteilen versehen war, um ein Gasgemisch zu- und abzuführen und Dauerversuche vorzunehmen. Die Versuchsdauer betrug jeweils 3 Wochen; danach wurden die innerhalb einer halben Stunde angefallenen bzw. aufgewendeten Massen bestimmt und Aktivität und Selektivität (vgl. Tabelle 2) errechnet.

### Neu-Katalysator

Ein Katalysator der in der Tabelle 1 angegebenen Zusammensetzung (vgl. EP 195 252) wurde in praktisch frischem Zustand untersucht.

### Versuch 1 (Vergleich)

Der Katalysator der in Versuch 1 angegebenen Zusammensetzung wurde ca. ein Jahr in einer Anlage unter technisch üblichen Bedingungen benutzt, ausgebaut und wie oben beschrieben untersucht (Zusammensetzung vgl. Tabelle 1).

### Versuch 2 (Vergleich)

Das benutzte Katalysatormaterial (vgl. Versuch 1) wurde auf die ursprüngliche Zusammensetzung des Neu-Katalysators gebracht und untersucht.

### Beispiel 1 (erfindungsgemäß)

Das benutzte Katalysatormaterial (vgl. Versuch 1) wurde zunächst auf 800°C erhitzt, um Ablagerungen zu entfernen und die Vermahlbarkeit zu verbessern, fein gemahlen, die in der Tabelle 1 angegebene Zusammensetzung eingestellt und wieder zu Strängen verformt.

**Tabelle 1**

| Chemische Zusammensetzung* der Katalysatoren [Gew.-%] | | | | | |
|---|---|---|---|---|---|
| | Fe₂O₃ | K₂O | WO₃ | Ce₂O₃ | CaO |
| Neu-Katalysator | 77,0 | 12,3 | 3,9 | 4,9 | 1,9 |
| Versuch 1 | 79,7 | 10,1 | 3,7 | 4,6 | 1,9 |
| Versuch 2 | 77,0 | 12,3 | 3,9 | 4,9 | 1,9 |
| Beispiel 1 | 75,5 | 11,2 | 3,7 | 7,6 | 2,0 |

| | | | | | |
|---|---|---|---|---|---|
| * Die Summe der Katalysatorbestandteile ergibt jeweils 100 % | | | | | |

**Tabelle 2**

| Eigenschaften der Katalysatoren (3 mm Stränge - Umsatz = 70 Mol-%) | | |
|---|---|---|
| | Selektivität (%) | Aktivität (°C) |
| Neu-Katalysator | 94,7 | 615 |
| Versuch 1 | 94,0 | 625 |
| Versuch 2 | 94,1 | 622 |
| Beispiel 1 | 94,9 | 615 |

## Patentansprüche

1. Verfahren zur Herstellung von Eisen, Kalium und Cer enthaltenden Katalysatoren für die Dehydrierung von Kohlenwasserstoffen aus ebensolchen gebrauchten Katalysatoren (Regenerierung) durch Vermahlen des gebrauchten Materials, gegebenenfalls Reinigung, Wiederherstellen der ursprünglichen Wirkung durch Einstellen der Zusammensetzung und Wiederherstellen der äußeren Form, dadurch gekennzeichnet, daß dem vermahlenen Material eine wirksame Menge an Kalium und eine solche Menge an Cer zugesetzt wird, daß die Gesamtmenge an Cer höher als die ursprünglich vorhandene Menge ist.

2. Verfahren nach Anspruch 1, dadurch gekennzeichnet, daß der Katalysator vor der Wiederverwendung calciniert wird.

3. Verfahren nach Anspruch 1, dadurch gekennzeichnet, daß der gebrauchte Katalysator vor dem Vermahlen in Gegenwart von Luft oder Sauerstoff calciniert wird.

## Claims

1. A process for the preparation of iron-, potassium- and cerium-containing catalysts for the dehydrogenation of hydrocarbons from the same spent catalysts (regeneration) by grinding and, if necessary, purifying the spent material, restoring the original activity by adjusting the composition and restoring the external shape, which comprises adding to the ground material an effective amount of potassium and such an amount of cerium that the total amount of cerium is greater than the amount originally present.

2. A process as claimed in claim 1, wherein the catalyst is calcined before re-use.

3. A process as claimed in claim 1, wherein the spent catalyst is calcined in the presence of air or oxygen before the grinding.

## Revendications

1. Procédé pour la préparation de catalyseurs contenant du fer, du potassium et du cérium pour la déshydrogénation d'hydrocarbures, à partir de ces mêmes catalyseurs usagés (régénération), par broyage fin de la matière usagée, par purification éventuelle, par rétablissement de l'activité initiale en réglant la composition et par rétablissement de la forme externe, caractérisé en ce qu'on ajoute à la matière moulue, une quantité efficace de potassium et une quantité de cérium telle que la quantité totale de cérium est supérieure à la quantité initialement présente.

2. Procédé selon la revendication 1, caractérisé en ce que le catalyseur est calciné avant la réutilisation.

3. Procédé selon la revendication 1, caractérisé en ce que le catalyseur usagé est calciné en présence d'air ou d'oxygène avant le broyage fin.
